# EUROPEAN PATENT APPLICATION

(11) **EP 4 350 377 A1**
(43) Date of publication of application: **10.04.2024**
(21) Application number: 22816100.6
(22) Date of filing: 31.05.2022
(51) Int. Cl.: G01R 33/12, A61B 5/0515, G01R 33/02, G01R 33/09

(54) **MAGNETISM MEASURING DEVICE**

(30) Priority: 02.06.2021 JP 2021093177
(71) Applicant: TDK Corporation, Tokyo 103-6128 (JP); NATIONAL UNIVERSITY CORPORATION YOKOHAMA NATIONAL UNIVERSITY, Yokohama-shi Kanagawa 240-8501 (JP)
(72) Inventor: KASAJIMA, Tamon, Tokyo 103-6128 (JP); OKAWA, Shuichi, Tokyo 103-6128 (JP); KOSUDA, Masanori, Tokyo 103-6128 (JP); TAKEMURA, Yasushi, Yokohama-shi, Kanagawa 240-8501 (JP); TRISNANTO, Suko Bagus, Yokohama-shi, Kanagawa 240-8501 (JP)
(74) Representative: Epping - Hermann - Fischer
(86) International application number: PCT/JP2022/022105
(87) International publication number: WO 2022/255354

(57) **Abstract**

To reduce the current value and frequency of a current flowing in an excitation coil in a magnetic field measurement device of a type that detects a magnetization change caused due to excitation of an object to be measured. A magnetic field measurement device 1 includes: an excitation coil C1 that applies an excitation AC magnetic field to an object to be measured including a magnetic material P to make a magnetization change of the magnetic material P exhibit linear response; a detection coil C0 that detects a primary detection AC magnetic field caused due to the magnetization change of the magnetic material P to generate a primary detection signal S1; a magnetic field generation coil C2 that generates a secondary detection AC magnetic field based on the primary detection signal S1; and a magnetic sensor 16 that detects the secondary detection AC magnetic field to generate a secondary detection signal S2 including a non-sine wave component. With this configuration, it is possible to obtain the secondary detection signal S2 including a non-sine wave component without making the magnetization change of the magnetic material P exhibit nonlinear response, allowing reduction in the current value and frequency of a current flowing in the excitation coil.

## Description

### [Technical Field]

The present invention relates to a magnetic field measurement device and, more particularly, to a magnetic field measurement device capable of being used for magnetic particle imaging.

### [Background Art]

There is known a magnetic particle imaging device (see Non-Patent Document 1) as a magnetic field measurement device of a type that detects a magnetization change caused due to excitation of an object to be measured. The magnetic particle imaging device is provided with an excitation coil that applies an excitation AC magnetic field to an object to be measured containing magnetic particles and a magnetic sensor that detects a detection AC magnetic field generated by the magnetization change of the excited magnetic particles. The magnetic sensor is applied not only with the detection AC magnetic field but also with the excitation AC magnetic field, and when the magnetization change of the magnetic particles is made to exhibit a nonlinear response, a detection signal component and an excitation component (noise component) included in an output signal from the magnetic sensor can be separated from each other through signal processing.

### [Citation List]

### [Non-Patent Document]

[Non-Patent Document 1] B. Gleich and J. Weizenecker, Nature, 435, 1214 (2005).

### [Disclosure of the Invention]

### [Problem to be Solved by the Invention]

However, in order to make the magnetization change of a magnetic material such as a magnetic particle exhibit nonlinear response, it is necessary to apply a very strong excitation AC magnetic field to an object to be measured. In addition, in order to detect the magnetization change made to exhibit nonlinear response, it is necessary to set the frequency of the excitation AC magnetic field to a high frequency of, e.g., 20 kHz or more. Therefore, when the size of an object to be measured is large to some extent, it is necessary to make an extremely large high-frequency current flow in the excitation coil, which, however, is impractical.

It is therefore an object of the present invention to reduce, in a magnetic field measurement device of a type that detects a magnetization change caused due to excitation of an object to be measured, the current value and frequency of a current flowing in an excitation coil.

### [Means for Solving the Problem]

A magnetic field measurement device according to the present invention includes: a first coil that applies an excitation AC magnetic field to an object to be measured including a magnetic material to make a magnetization change of the magnetic material exhibit linear response; a first magnetic sensor that detects a primary detection AC magnetic field caused due to the magnetization change of the magnetic material to generate a primary detection signal; a second coil that generates a secondary detection AC magnetic field based on the primary detection signal; and a second magnetic sensor that detects the secondary detection AC magnetic field to generate a secondary detection signal including a non-sine wave component.

According to the present invention, the primary detection signal generated by the first magnetic sensor is converted back to a magnetic field, and the obtained magnetic field is detected by the second magnetic sensor, so that it is possible to obtain the secondary detection signal including a non-sine wave component without making the magnetization change of the magnetic material exhibit nonlinear response. This allows high-sensitivity detection of the magnetization change of the magnetic material even though the current value of a current flowing in the first coil is small and the frequency thereof is low.

The magnetic field measurement device according to the present invention may further include a third coil that cancels the excitation AC magnetic field applied to the first magnetic sensor. This makes it possible to reduce a noise component included in the primary detection signal.

The magnetic field measurement device according to the present invention may further include a signal processing circuit that detects a harmonic component included in the secondary detection signal. This makes it possible to remove a noise component included in the secondary detection signal.

### [Advantageous Effects of the Invention]

As described above, according to the present invention, in a magnetic field measurement device of a type that detects a magnetization change caused due to excitation of an object to be measured, the current value and frequency of a current flowing in an excitation coil can be reduced.

### [Brief Description of the Drawings]

FIG. 1 is a schematic diagram for explaining the configuration of a magnetic field measurement device 1 according to an embodiment of the present invention.
FIG. 2 is a schematic view for explaining the magnetization change of the magnetic material P.
FIG. 3 is a circuit diagram of the magnetic sensor 16.
FIG. 4 is a schematic graph for explaining the magnetization change of the magnetic material P, in which the vertical and horizontal axes represent a magnetization M and a magnetic field H, respectively.
FIG. 5 is a schematic graph for explaining a change in the secondary detection signal S2, in which the vertical and horizontal axes represent a voltage V and a magnetic field H, respectively.
FIGS 6A to 6H are graphs each illustrating a signal waveform. FIG. 6A illustrates the waveform of the excitation AC current i1, FIG. 6B illustrates the waveform of the primary detection signal S1, FIG. 6C illustrates the waveform of the secondary detection signal S2, FIGS. 6D to 6H respectively illustrate the waveforms of a third harmonic, a fifth harmonic, a seventh harmonic, a ninth harmonic, and an eleventh harmonic, which are contained in the secondary detection signal S2.

### [Mode for Carrying Out the Invention]

Hereinafter, preferred embodiments of the present invention will be described in detail with reference to the accompanying drawings.

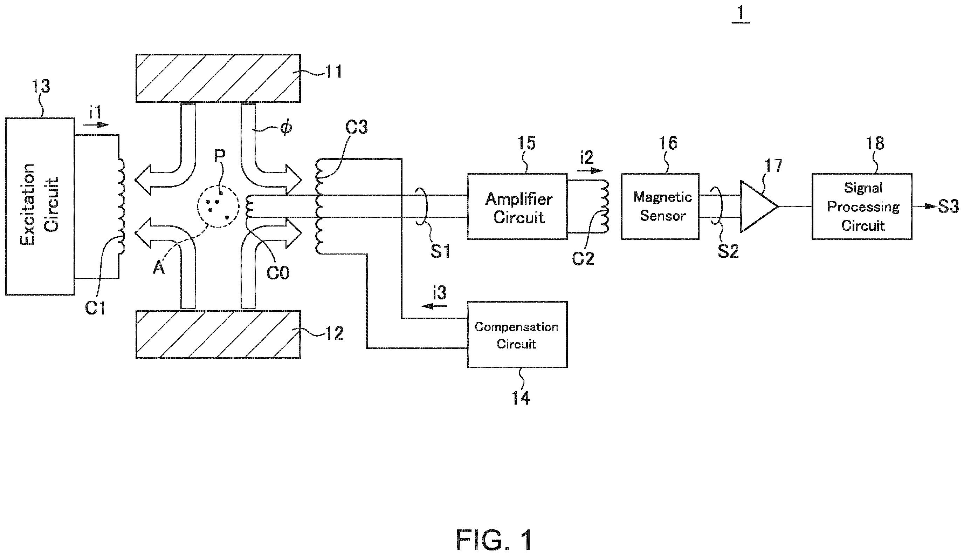

FIG. 1 is a schematic diagram for explaining the configuration of a magnetic field measurement device 1 according to an embodiment of the present invention.

The magnetic field measurement device 1 according to the present embodiment is a device that detects a magnetic material P in an object to be measured positioned in a measurement area A and includes, as illustrated in FIG. 1, magnets 11 and 12 that generate a gradient DC magnetic field φ, an excitation circuit 13 connected to an excitation coil C1, and a detection coil C0 that detects a primary detection AC magnetic field caused due to a magnetization change of the magnetic material P. The magnetic material P may be a magnetic nanoparticle with nanosize. Using the magnetic nanoparticle as the magnetic material P allows a human body to be set as the object to be measured.

The magnets 11 and 12 are arranged such that the S-poles or N-poles thereof face each other so as to substantially null the strength of the gradient DC magnetic field φ in the measurement area A. Coils may be used in place of the magnets 11 and 12. Further alternatively, a mechanism that spatially moves the measurement area A may be provided. The excitation circuit 13 is a circuit for making an excitation AC current i1 flow in the excitation coil C1, whereby an excitation AC magnetic field is applied to the measurement area A. The excitation AC current i1 has a sine waveform. As described later, the strength of the excitation AC magnetic field is set to a value making the magnetization change of the magnetic material P positioned in the measurement area A exhibit nonlinear response. The nonlinear response means that the magnetic material P undergoes a magnetization change in an unsaturated region. Thus, the nonlinear response may include not only a case where the magnetization change is completely linear, but also a case where it includes some nonlinear component so long as the magnetization change occurs in the unsaturated region.

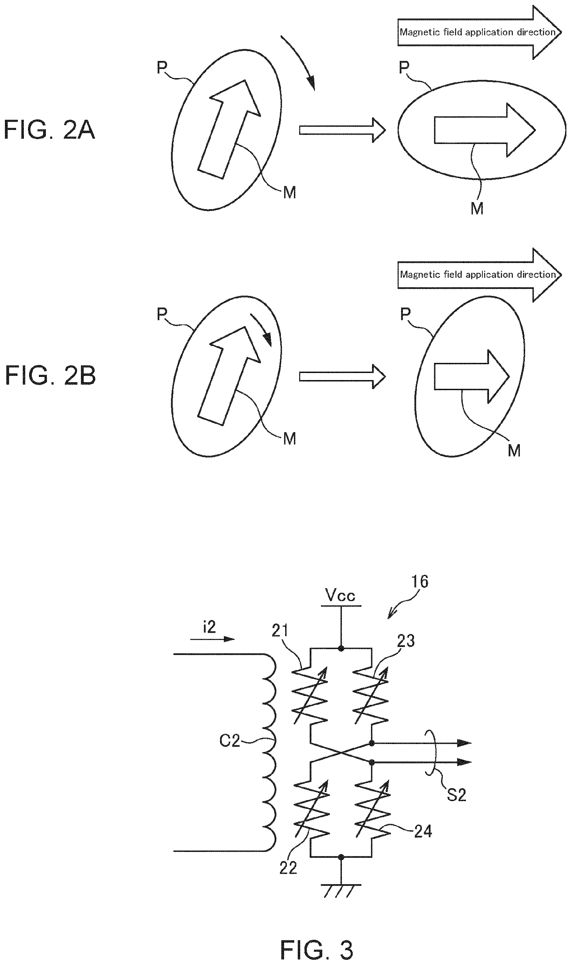

FIG. 2 is a schematic view for explaining the magnetization change of the magnetic material P.

In the example illustrated in FIG. 2A, when an excitation magnetic field is applied to the magnetic material P, the magnetic material P itself, which has a magnetization M in a predetermined direction, rotates, with the result that the direction of the magnetization M changes. In the example illustrated in FIG. 2B, when an excitation magnetic field is applied to the magnetic material P, the magnetization M inside the magnetic material P rotates. In either case, application of the excitation magnetic field to the magnetic material P causes a change in the direction of the magnetization M of the magnetic material P. Further, both the rotation of the magnetic material P itself illustrated in FIG. 2A and rotation of the magnetization M inside the magnetic material P illustrated in FIG. 2B may occur by application of the excitation magnetic field. In the present embodiment, a change in the direction of the magnetization M caused by application of the excitation magnetic field to the magnetic material P is defined as "magnetization change".

The magnetization change of the magnetic material P generates a primary detection AC magnetic field. The primary detection AC magnetic field is detected by the detection coil C0 as a first magnetic sensor, and a primary detection signal S1 is generated. In the present embodiment, the above detection coil C0 is used as a magnetic sensor for detecting the primary detection AC magnetic field; however, the magnetic sensor for detecting the primary detection AC magnetic field is not limited to this, but a magnetic sensor using a magnetosensitive element may be employed. Although the excitation AC magnetic field is also applied to the magnetic material P existing outside the measurement area A, the magnetization change does not substantially occur since the direction of the magnetization M is fixed by the gradient DC magnetic field φ having a predetermined strength at the outside of the measurement area A. This allows the detection coil C0 to selectively detect the magnetization change of the magnetic material P positioned within the measurement area A.

The excitation AC magnetic field applied to the detection coil C0 is canceled by a cancel coil C3. A cancel current i3 is made to flow in the cancel coil C3 by a compensation circuit 14, whereby the excitation AC magnetic field applied to the detection coil C0 is canceled. However, it is difficult to completely cancel the excitation AC magnetic field applied to the detection coil C0 and, thus, a slight noise component originating from the excitation AC magnetic field is superimposed on the primary detection signal S1.

The primary detection signal S1 is input to an amplifier circuit 15. The amplifier circuit 15 is an analog circuit including a differential amplifier and a filter circuit and supplies a detection AC current i2 to a magnetic field generation coil C2 based on the primary detection signal S1. As a result, a secondary detection AC magnetic field is generated from the magnetic field generation coil C2. The amplifier circuit 15 has substantially no delay since it is an analog circuit, so that a secondary detection AC magnetic field is generated in substantially real time in accordance with the primary detection AC magnetic field. The secondary detection AC magnetic field is detected by a second magnetic sensor 16, and a secondary detection signal S2 is generated.

FIG. 3 is a circuit diagram of the magnetic sensor 16.

As illustrated in FIG. 3, the magnetic sensor 16 is constituted by magnetosensitive elements 21 to 24 which are full-bridge connected. The magnetosensitive elements 21 to 24 may be an element having high sensitivity even in a low frequency region and capable of being magnetically saturated, examples thereof including: magnetoresistive effect elements such as a TMR (Tunnel Magneto-Resistive effect) element, a GMR (Giant Magneto-Resistive effect) element, and an AMR (Anisotropic Magneto-Resistive effect) element; a hall element; and an MI (Magnetic Impedance) element. The magnetic sensor 16 is configured such that the secondary detection AC magnetic field generated from the magnetic field generation coil C2 is applied in mutually opposite directions to the magnetosensitive elements 21, 22 and magnetosensitive elements 23, 24. As a result, the secondary detection signal S2 corresponding to the secondary detection AC magnetic field is output from the magnetic sensor 16. The magnetic sensor 16 may be not only a sensor obtained by full-bridge connecting four magnetosensitive elements but also a sensor obtained by half-bridge connecting two magnetosensitive elements and a sensor using a single magnetosensitive element.

The secondary detection signal S2 is supplied to a signal processing circuit 18 through an amplifier 17. The signal processing circuit 18 extracts a harmonic component included in the secondary detection signal S2 to generate a tertiary detection signal S3. The tertiary detection signal S3 is a final output signal of the magnetic field measurement device 1 according to the present embodiment and indicates the magnetization change of the magnetic material P positioned in the measurement area A. When an imaging device for imaging the obtained tertiary detection signal S3 is combined with the thus configured magnetic field measurement device 1, a magnetic particle imaging device can be constituted.

The configuration of the magnetic field measurement device 1 according to the present embodiment has been described. The following describes the operation of the magnetic field measurement device 1 according to the present embodiment.

The excitation circuit 13 makes the excitation AC current i1 flow in the excitation coil C1 such that the magnetization change of the magnetic material P positioned in the measurement area A exhibits linear response. That is, the amount of the excitation AC current i1 supplied to the excitation coil C1 is set to a value sufficiently smaller than an amount required for making the magnetization change of the magnetic material P exhibit nonlinear response.

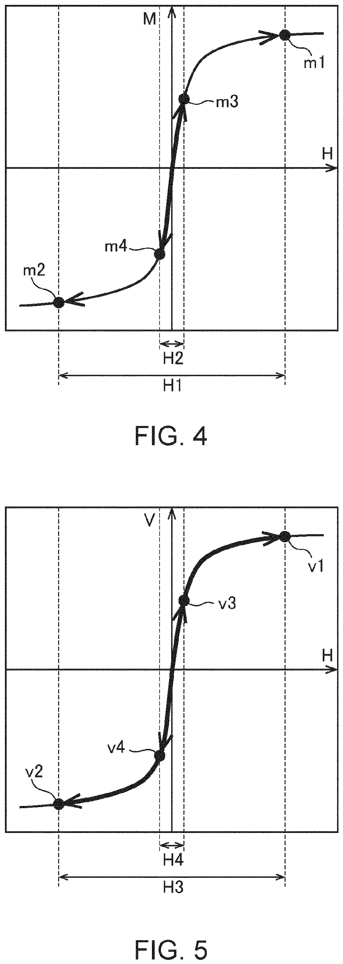

FIG. 4 is a schematic graph for explaining the magnetization change of the magnetic material P, in which the vertical and horizontal axes represent a magnetization M and a magnetic field H, respectively.

As illustrated in FIG. 4, when the amplitude of the magnetic field H is set to H1, the magnetization M of the magnetic material P is saturated to exhibit nonlinear response between the magnetization m1 and the magnetization m2 in the graph. In this case, a detection signal component included in the primary detection signal S1 assumes a non-sine wave. When the object to be measured has a size comparable to a human body, a magnetic field as strong as about 6 mT is required in order to make the magnetization M of the magnetic material P as a magnetic nanoparticle exhibit nonlinear response. On the other hand, when the amplitude of the magnetic field H is set to H2 (< H1), the magnetization M of the magnetic material P changes in the unsaturated region and thus exhibits linear response between the magnetization m3 and the magnetization m4 in the graph. In this case, the detection signal component included in the primary detection signal S1 is presented by a sine wave.

As described above, the amount of the excitation AC current i1 is reduced to an amount with which the magnetization change of the magnetic material P exhibits linear response, so that as compared with a case where the magnetization change of the magnetic material P exhibits nonlinear response, the amount of the excitation AC current i1 is significantly reduced. When the object to be measured has a size comparable to a human body, it is sufficient to set a magnetic field to, e.g., 0.1 mT in order to make the magnetization M of the magnetic material P as a magnetic nanoparticle exhibit linear response. That is, the amount of the excitation AC current i1 is reduced to 1/10 or less of the amount required for making the magnetization change of the magnetic material P exhibit nonlinear response.

As described above, in the present embodiment, the magnetization change of the magnetic material P is made to exhibit linear response by the excitation coil C1, so that, in the primary detection signal S1 generated by the detection coil C0, the detection signal component originating from the primary detection AC magnetic field is presented by a sine wave. The primary detection signal S1 includes also a noise component originating from the excitation AC magnetic field that has not been canceled completely. However, this noise component is reduced to a sufficiently low level by the cancel coil C3, and thus the detection signal component is dominant. The primary detection signal S1 is converted to the detection AC current i2 by the amplifier circuit 15, whereby the secondary detection AC magnetic field is generated from the magnetic field generation coil C2. The secondary detection AC magnetic field is detected by the magnetic sensor 16, and the secondary detection signal S2 is generated.

FIG. 5 is a schematic graph for explaining a change in the secondary detection signal S2, in which the vertical and horizontal axes represent a voltage V and a magnetic field H, respectively.

As illustrated in FIG. 5, the amplitude of a detection signal component included in the secondary detection AC magnetic field is H3. The magneto-resistive effect of the magnetosensitive elements 21 to 24 is saturated with respect to a component having an amplitude of H3 in the secondary detection AC magnetic field, with the result that the voltage V of the secondary detection signal S2 exhibits nonlinear response between the voltage v1 and the voltage v2 in the graph. In this case, the detection signal component included in the secondary detection signal S2 assumes a non-sine wave. On the other hand, a noise component included in the secondary detection AC magnetic field is H4 (< H3). The magnetosensitive elements 21 to 24 operate in the unsaturated region with respect to a component having an amplitude of H4 in the secondary detection AC magnetic field, with the result that the voltage V of the secondary detection signal S2 exhibits linear response between the voltage v3 and the voltage v4 in the graph.

In order to make the magnetosensitive elements 21 to 24 exhibit nonlinear response based on the detection signal component and to make the magnetosensitive elements 21 to 24 exhibit linear response based on the noise component, a preliminary magnetic field measurement operation is performed in a state where the magnetic material P does not exist in the measurement area A, i.e., a state where no detection signal component is included, and then the gain of the amplifier circuit 15 and filter characteristics are adjusted so as to make the magnetosensitive elements 21 to 24 exhibit linear response based on the noise component originating from the excitation AC magnetic field.

As a result, the detection signal component included in the secondary detection AC magnetic field is converted to a non-sine wave component of the secondary detection signal S2, while the noise component included in the secondary detection AC magnetic field is converted to a sine wave component of the secondary detection signal S2. That is, although both the detection signal component and noise component included in the primary detection signal S1 assume a sine wave, they are separated into a non-sine wave component and a sine wave component, respectively, through conversion into a magnetic field using the magnetic field generation coil C2 and additional conversion into the secondary detection signal S2 using the magnetic sensor 16.

The thus generated secondary detection signal S2 is supplied to the signal processing circuit 18 through the amplifier 17. The signal processing circuit 18 extracts a harmonic component included in the secondary detection signal S2 to generate a tertiary detection signal S3. As described above, the detection signal component included in the secondary detection signal S2 is a non-sine wave component, so that a harmonic occurs. On the other hand, the noise component included in the secondary detection signal S2 is a sine wave component, a harmonic hardly occurs. Thus, by detecting a harmonic component included in the secondary detection signal S2, it is possible to selectively extract the detection signal component.

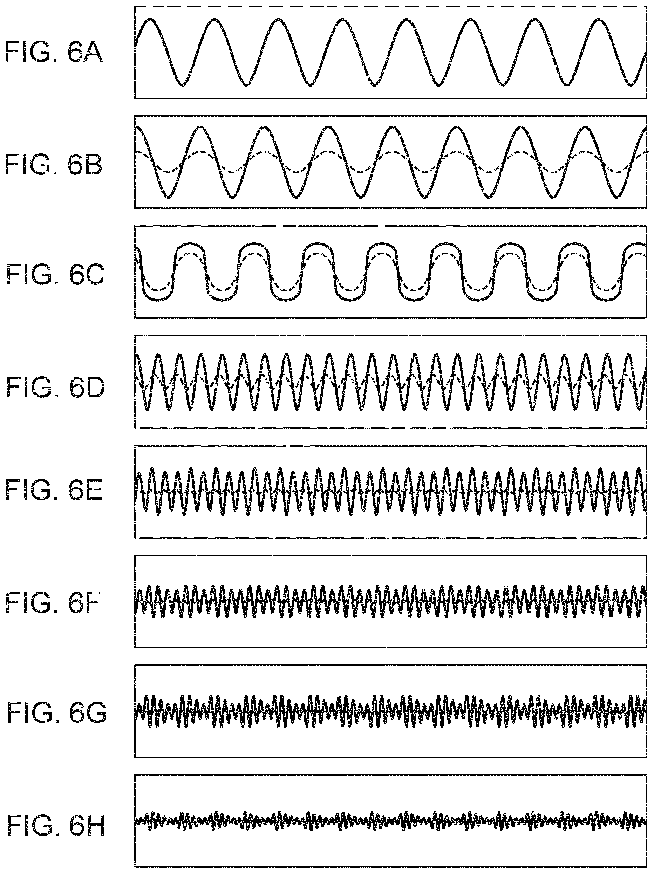

FIGS 6A to 6H are graphs each illustrating a signal waveform. FIG. 6A illustrates the waveform of the excitation AC current i1, FIG. 6B illustrates the waveform of the primary detection signal S1, FIG. 6C illustrates the waveform of the secondary detection signal S2, FIGS. 6D to 6H respectively illustrate the waveforms of a third harmonic, a fifth harmonic, a seventh harmonic, a ninth harmonic, and an eleventh harmonic, which are contained in the secondary detection signal S2. In FIGS. 6B to 6H, the solid line denotes the detection signal component, and the dashed line denotes the noise component.

As illustrated in FIG. 6A, the excitation AC current i1 assumes a sine wave. In the present embodiment, since the magnetization M of the magnetic material P is made to exhibit linear response by the excitation AC magnetic field, both the detection signal component and noise component included in the primary detection signal S1 assume a sine wave, as illustrated in FIG. 6C. However, the detection signal component is converted to a non-sine wave using the magnetic field generation coil C2 and magnetic sensor 16, so that, as illustrated in FIG. 6C, the detection signal component included in the secondary detection signal S2 assumes a non-sine wave, while the noise component included in the secondary detection signal S2 assumes a sine wave. As a result, as illustrated in FIGS. 6D to 6H, a large harmonic appears in the detection signal component, while substantially no harmonic appears in the noise component. For example, when the ratio (SN ratio) between the detection signal component and the noise component included in the primary detection signal S1 is 9.6 dB, the SN ratios of the respective third harmonic, fifth harmonic, seventh harmonic, ninth harmonic, and eleventh harmonic contained in the secondary detection signal S2 are 11.1 dB, 15.3 dB, 14.7 dB, 13.3 dB, and 9.0 dB, respectively.

Thus, by extracting a predetermined harmonic component from the secondary detection signal S2 using the signal processing circuit 18, it is possible to take out the detection signal component caused due to magnetization change of the magnetic material P. The thus extracted detection signal component is output outside as a tertiary detection signal S3.

As described above, the magnetic field measurement device 1 according to the present embodiment makes the magnetization M of the magnetic material P exhibit linear response based on the excitation AC magnetic field, while it selectively converts the detection signal component to a non-sine wave using the magnetic field generation coil C2 and magnetic sensor 16, so that it is possible not only to significantly reduce the amount of the excitation AC current i1 but also to ensure a sufficient SN ratio even though the frequency of the excitation AC current i1 is reduced to about 10 kHz. In addition, the primary detection signal S1 is converted to the secondary detection signal S2 using a physical device, thus preventing a delay that may occur when the primary detection signal S1 is directly subjected to signal processing. This enables magnetic particle imaging with respect to an object to be measured having a relatively large size like a human body.

While the preferred embodiment of the present disclosure has been described, the present disclosure is not limited to the above embodiment, and various modifications may be made within the scope of the present disclosure, and all such modifications are included in the present disclosure.

### [Reference Signs List]

- 1: magnetic field measurement device
- 11, 12: magnet
- 13: excitation circuit
- 14: compensation circuit
- 15: amplifier circuit
- 16: magnetic sensor
- 17: amplifier
- 18: signal processing circuit
- 21-24: magnetosensitive element
- A: measurement area
- C0: detection coil
- C1: excitation coil
- C2: magnetic field generation coil
- C3: cancel coil
- P: magnetic material
- S1: primary detection signal
- S2: secondary detection signal
- S3: tertiary detection signal
- i1: excitation AC current
- i2: detection AC current
- i3: cancel current
- φ: gradient DC magnetic field

## Claims

1. A magnetic field measurement device comprising:
a first coil that applies an excitation AC magnetic field to an object to be measured including a magnetic material to make a magnetization change of the magnetic material exhibit linear response;
a first magnetic sensor that detects a primary detection AC magnetic field caused due to the magnetization change of the magnetic material to generate a primary detection signal;
a second coil that generates a secondary detection AC magnetic field based on the primary detection signal; and
a second magnetic sensor that detects the secondary detection AC magnetic field to generate a secondary detection signal including a non-sine wave component.

2. The magnetic field measurement device as claimed in claim 1, further comprising a third coil that cancels the excitation AC magnetic field applied to the first magnetic sensor.

3. The magnetic field measurement device as claimed in claim 1 or 2, further comprising a signal processing circuit that detects a harmonic component included in the secondary detection signal.
